# EUROPEAN PATENT APPLICATION

(11) **EP 2 902 118 A2**
(43) Date of publication of application: **05.08.2015**
(21) Application number: 15153030.0
(22) Date of filing: 29.01.2015
(51) Int. Cl.: B06B 1/06

(54) **Ultrasonic device, probe, electronic device, and ultrasound imaging apparatus**

(30) Priority: 31.01.2014 JP 2014016639
(71) Applicant: Seiko Epson Corporation, Tokyo 163-0811 (JP)
(72) Inventor: Kiyose, Kanechika, Nagano, 392-8502 (JP)
(74) Representative: Hoffmann Eitle

(57) **Abstract**

An ultrasonic device (17) includes a substrate (21), an acoustic matching layer, an acoustic lens (52, 81, 87), and a structure (53, 79). The substrate (21) has an element array (22) including a plurality of thin-film ultrasonic transducer elements (23) arranged in an array form. The acoustic matching layer covers the element array (22). The acoustic lens (52, 81, 87) is arranged on the acoustic matching layer. The structure (53, 79) is arranged between the acoustic lens (52, 81, 87) and the substrate (21) and has a larger compressive strength than a compressive strength of the acoustic matching layer.

## Description

### BACKGROUND

### Technical Field

The present invention relates to an ultrasonic device, and a probe, an electronic device, an ultrasound imaging apparatus that use the same, and a manufacturing method of an ultrasonic device.

### Related Art

As disclosed in Japanese Laid-Open Patent Publication No. 2008-193357, an ultrasound diagnostic apparatus called an ultrasound imaging apparatus is commonly known. An ultrasound imaging apparatus is provided with a plurality of ultrasonic transducer elements arranged in an array form. The ultrasonic transducer elements are formed from so-called capacitive micromachined ultrasonic transducers (cMUTs; electrostatic capacitive type). An acoustic lens covers the array of ultrasonic transducer elements. The acoustic lens is affixed to the array of ultrasonic transducers by an adhesive agent. The acoustic lens is formed from silicone rubber.

### SUMMARY

An acoustic lens that matches a living body has an acoustic impedance close to that of the living body. Consequently, the acoustic lens has the same degree of softness as the living body. When the acoustic lens is pressed against the living body, the pressing force toward the array of ultrasonic transducer elements acts on the acoustic lens. The acoustic lens is compressed. When this acoustic lens deforms, an offset is produced in the focus position of the acoustic lens. A clear ultrasound image cannot be obtained.

An ultrasonic transducer capable of suppressing the deformation of the acoustic lens when pressed against a living body was desired.
(1) An ultrasonic device according to one aspect includes a substrate, an acoustic matching layer, an acoustic lens, and a structure. The substrate has an element array including a plurality of thin-film ultrasonic transducer elements arranged in an array form. The acoustic matching layer covers the element array. The acoustic lens is arranged on the acoustic matching layer. The structure is arranged between the acoustic lens and the substrate and has a larger compressive strength than a compressive strength of the acoustic matching layer.

When the acoustic lens is pressed against the living body, the pressing force toward the substrate acts on the acoustic lens. At this time, the acoustic lens is stopped by the structure in the direction of the pressing force because the structure has larger compressive strength than the compressive strength of the acoustic matching layer. Thus, deformation of the acoustic lens is prevented even when a pressing force is applied to this acoustic lens. Deformation of the acoustic lens is adequately suppressed for a pressing force in the ultrasonic device.
(2) The structure preferably sandwiches the acoustic matching layer by two side surfaces in contact respectively with side surfaces of the acoustic matching layer extending along a direction parallel to a generatrix of the acoustic lens. A partial cylindrical surface that is formed by a generatrix parallel to one center line is defined on the acoustic lens. The partial cylindrical surface is used to converge ultrasonic waves. The focus position of the ultrasonic waves is determined by the partial cylindrical surface. The acoustic lens is supported by the structure along the outline parallel to the generatrix of the acoustic lens because the side surfaces of the structure sandwich the acoustic matching layer in the direction that intersects the generatrix. Generally, the acoustic lens is longer in the direction parallel to the generatrix compared to the direction that intersects the generatrix. Therefore, the gap between a pair of structures sandwiching the acoustic matching layer is sandwiched. Thus, deformation of the acoustic lens is effectively suppressed.
(3) The structure preferably sandwiches the acoustic matching layer by two side surfaces in contact respectively with side surfaces of the acoustic matching layer extending along a direction intersecting the generatrix of the acoustic lens. Here, the acoustic lens is supported by the structure along the outline in the direction intersecting the generatrix of the acoustic lens because the side surfaces of the structure sandwich the acoustic matching layers in the direction along the generatrix of the acoustic lens. Thus, deformation of the acoustic lens is more effectively suppressed.
(4) The structure preferably encloses the acoustic matching layer along a surface of the substrate. Crushing of the acoustic matching layer is prevented by the structure even when a pressing force acts on the acoustic matching layer. Deformation of the acoustic matching layer is prevented. The acoustic lens is effectively supported by the structure and the acoustic matching layer. Deformation of the acoustic lens is suppressed.
(5) The acoustic lens preferably has a columnar part projecting out toward the substrate from a surface facing the substrate, and the acoustic matching layer preferably has a through hole having an inner surface in contact with the columnar part. According to this structure, offsets of the acoustic lens and the acoustic matching layer are prevented in the shear direction along the boundary surface of the acoustic lens and the acoustic matching layer. The acoustic lens and the acoustic matching layer are firmly stacked to construct a layered structure. This layered structure further enhances the suppression of deformation of the acoustic lens.
(6) The ultrasonic device preferably further includes a flexible wiring board bonded to the substrate on an outside of an outline of the acoustic matching layer in a plan view along a thickness direction of the substrate, and the structure preferably covers a conductor on the substrate between the acoustic matching layer and the flexible wiring board. The element array and the flexible printed wiring board are electrically connected to each other by the conductor on the substrate. Exposure of the conductor is prevented because the conductor is covered by the structure between the acoustic matching layer and the flexible printed wiring board. The conductor on the substrate is protected.
(7) The structure is preferably arranged on the flexible printed wiring board. The structure can reinforce the fixing strength of the flexible printed wiring board.
(8) The ultrasonic device can be used in a probe. This probe may be provided with the ultrasonic device and a case that supports the ultrasonic device.
(9) In the probe, the structure is preferably fixed to the case. The ultrasonic device is housed in the case during the manufacture of the probe. The structure in the case prevents movement of the ultrasonic device. The acoustic lens is reliably fixed to the case.
(10) The ultrasonic device can be used in an electronic device. The electronic device may be provided with an ultrasonic device and a processing unit that is connected to the ultrasonic device and processes the output of the ultrasonic device.
(11) The ultrasonic device can be used in an ultrasound imaging apparatus. The ultrasound imaging apparatus may be provided with an ultrasonic device; a processing unit that is connected to the ultrasonic device, processes the output of the ultrasonic device, and generates an image; and a display device that displays the image.
(12) An ultrasonic device according to another aspect includes a substrate, an acoustic matching layer, and an acoustic lens. The substrate has an element array including a plurality of thin-film ultrasonic transducer elements arranged in an array form. The acoustic matching layer covers the element array and has a first compressive strength. The acoustic lens is arranged on the acoustic matching layer and has a second compressive strength that is smaller than the first compressive strength.

When an acoustic lens is pressed against a living body, the pressing force directed toward the substrate acts on the acoustic lens. The acoustic lens is stopped in the direction of the pressing force by the acoustic matching layer. Crushing of the acoustic matching layer is prevented compared to when the acoustic matching layer has a compressive strength equivalent to that of the acoustic lens. Deformation of the acoustic lens can be prevented even when the pressing force is applied to the acoustic lens. Deformation of the acoustic lens can be adequately suppressed for a pressing force in the ultrasound device.
(13) The acoustic matching layer is preferably a single layer. As a result, the formation step of the acoustic matching layer can be simplified compared to when the acoustic matching layer is formed from a plurality of layers. Moreover, the film thickness of the acoustic matching layer can be controlled with high precision.
(14) An acoustic impedance of the acoustic matching layer is preferably 2 MRayls or less. Matching of the acoustic impedance between the acoustic lens and the thin-film transducer elements can be established. For example, the acoustic matching layer is formed into a thinner film compared to bulk transducer elements.
(15) A film thickness of the acoustic matching layer is preferably 100 µm or less. Deformation of the acoustic matching layer is suppressed in response to the thinning of the film. Moreover, the ultrasonic device is reduced in size.
(16) A manufacturing method of an ultrasonic device according to another aspect includes: arranging a masking material on a substrate having an element array including a plurality of thin-film ultrasonic transducer elements arranged in an array form so that the masking material is arranged on both sides of a region of the substrate where the element array is arranged in a plan view along a thickness direction of the substrate; arranging an acoustic matching layer and an acoustic lens on the element array between the masking material so that the masking material is sandwiched between the acoustic lens and the substrate; removing the masking material; and forming a structure between the acoustic lens and the substrate, the structure having a larger compressive strength than a compressive strength of the acoustic matching layer. Thus, it is possible to manufacture the ultrasonic device described above.
(17) The manufacturing method of an ultrasonic device preferably further includes bonding a flexible printed wiring board to the substrate at a position between an edge of the region and an edge of the substrate on both sides of the region, after the removing of the masking material. The forming of the structure preferably includes pouring a resin material having fluidity between the acoustic matching layer and the flexible printed wiring boards and curing the resin material to form the structure.

The element array and a flexible printed wiring board are electrically connected to each other by a conductor on the substrate. Exposure of the conductor is avoided by covering the conductor between the acoustic matching layer and the flexible printed wiring board with a structure. Resin material is poured into the spaces between the acoustic matching layer and the flexible printed wiring board in the formation of the structure. The resin material can reliably cover the conductor. On the other hand, when dripped resin material is pressed to spread out by another part and the resin material fills the space, the resin material cannot adequately spread into the corners in the space.
(18) In the manufacturing method of the ultrasonic device, the arranging of the acoustic lens preferably includes positioning the acoustic lens using the masking material contacting the acoustic lens from both sides of the acoustic lens. The acoustic lens can be positioned with high precision with respect to the element array by the function of the masking material.
(19) In the manufacturing method of the ultrasonic device, the arranging of the acoustic lens preferably includes positioning the acoustic lens using the masking material having an opening in which the acoustic lens is placed so that the masking material positions the acoustic lens in four directions. The acoustic lens can be positioned with high precision with respect to the element array by the action of the masking material.
(20) In the manufacturing method of the ultrasonic device, the arranging of the acoustic matching layer preferably includes pouring resin material having fluidity into the opening, and controlling a thickness of the resin material by a thickness of the masking material. The opening delineates a frame that encloses the element array on the substrate. Resin material in the acoustic lens is poured into the frame. The flow of the resin material is dammed. Thus, the shape of this acoustic matching layer is constructed; and the thickness of the acoustic lens can be determined.

### BRIEF DESCRIPTION OF THE DRAWINGS

Referring now to the attached drawings which form a part of this original disclosure:
Fig. 1 is an exterior diagram schematically showing an example of an electronic device related to an embodiment, namely an ultrasound diagnostic apparatus.
Fig. 2 is an enlarged planar diagram of an ultrasonic probe.
Fig. 3 is an enlarged planar diagram of an ultrasonic device related to the first embodiment.
Fig. 4 is a cross-sectional diagram along line A-A in Fig. 3.
Fig. 5 is a perspective diagram of an ultrasonic device.
Fig. 6 is a cross-sectional diagram along line B-B in Fig. 5.
Fig. 7 is a diagram showing a manufacturing method of an ultrasonic device and is an enlarged cross-sectional diagram that schematically shows the masking material formed on the substrate.
Fig. 8 is a diagram showing a manufacturing method of an ultrasonic device and is a partial enlarged cross-sectional diagram that schematically shows resin material poured on an element array.
Fig. 9 is a diagram showing a manufacturing method of an ultrasonic device and is a partial enlarged cross-sectional diagram that schematically shows an acoustic lens arranged in the opening of the masking material.
Fig. 10 is a diagram showing a manufacturing method of an ultrasonic device and is a partial enlarged cross-sectional diagram that schematically shows a first wiring board and a second wiring board that are bonded to the substrate after the masking material was removed.
Fig. 11 is a diagram showing a manufacturing method of an ultrasonic device and is a partial enlarged cross-sectional diagram that schematically shows a protective film formed on the substrate.
Fig. 12 is a vertical cross-sectional diagram that schematically shows an ultrasonic device related to the first modified example.
Fig. 13 is a cross-sectional diagram that schematically shows an ultrasonic device related to the second modified example corresponding to Fig. 4.
Fig. 14 is a cross-sectional diagram that schematically shows an ultrasonic device related to the third modified example corresponding to Fig. 4.

### DETAILED DESCRIPTION OF EXEMPLARY EMBODIMENTS

An embodiment of the present invention is explained with reference to the attached drawings. The embodiment explained below does not unreasonably limit the content of the present invention described in the scope of the patent claims, and does not limit any of the structures described in this embodiment that are essential as means for solving the present invention.

### (1) Overall Configuration of Ultrasound Imaging Apparatus

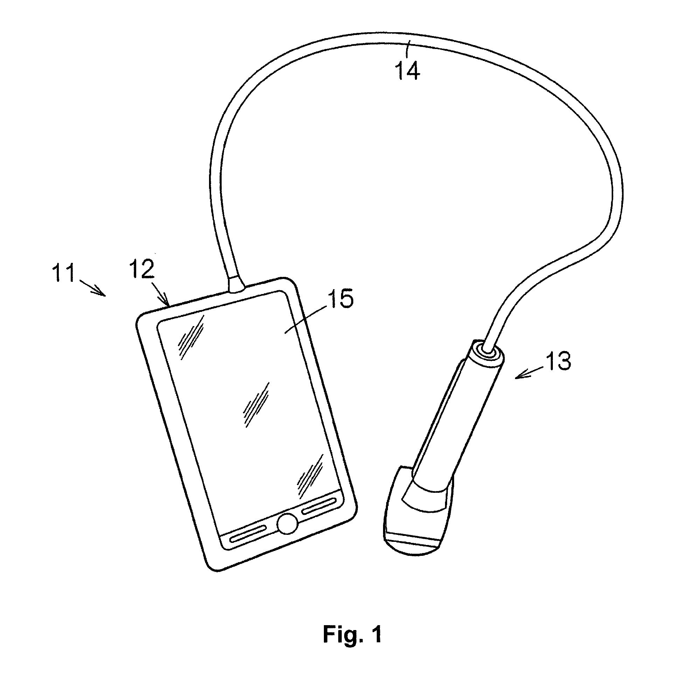

Fig. 1 schematically shows an example of an electronic device related to one embodiment of the present invention, namely the configuration of an ultrasound diagnostic apparatus (ultrasound imaging apparatus) 11. The ultrasound diagnostic apparatus 11 is provided with an apparatus terminal (processing unit) 12 and an ultrasonic probe (probe) 13. The apparatus terminal 12 and the ultrasonic probe 13 are connected to each other by a cable 14. The apparatus terminal 12 and the ultrasonic probe 13 exchange electrical signals through the cable 14. A display panel (display device) 15 is incorporated into the apparatus terminal 12. The screen of the display panel 15 is exposed by the screen of the apparatus terminal 12. In the apparatus terminal 12, an image is generated based on ultrasonic waves detected by the ultrasonic probe 13. The imaging of the detection result is displayed on the screen of the display panel 15.

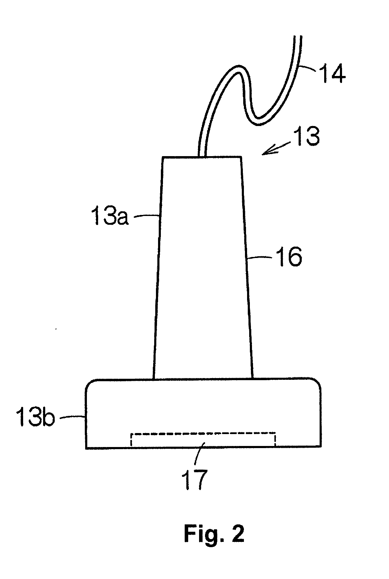

As shown in Fig. 2, the ultrasonic probe 13 has a case 16. An ultrasonic device 17 is housed in the case 16. The front surface of the ultrasonic device 17 can be exposed at the front screen of the case 16. The ultrasonic device 17 outputs ultrasonic waves from the front surface thereof and receives the reflected waves of the ultrasonic waves. In addition, the ultrasonic probe 13 can be provided with a probe head 13b that is detachably connected to a probe body 13a. The ultrasonic device 17 can be incorporated into the case 16 of the probe head 13b.

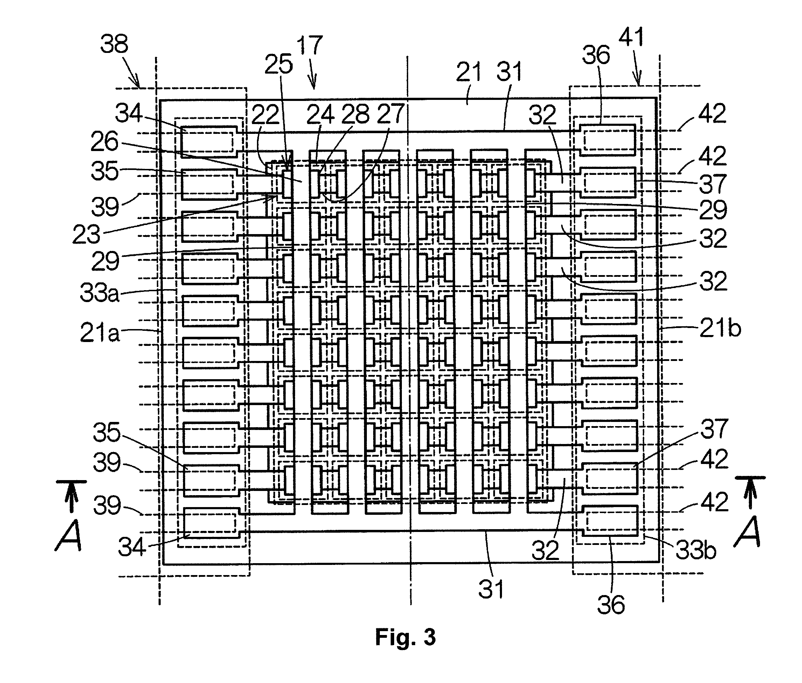

Fig. 3 schematically shows a planar diagram of the ultrasonic device 17. The ultrasonic device 17 is provided with a substrate (base material) 21. An element array 22 is formed in the substrate 21. The element array 22 is composed of an array of ultrasonic transducer elements 23 (hereinafter, referred to as the "elements") arranged in an array form. The array is formed by a matrix of a plurality of rows and a plurality of columns. In addition, a staggered arrangement may be established in the array. The group of elements 23 in the even rows in the staggered arrangement may be staggered by one-half of the row pitch with respect to the group of elements 23 in the odd rows. The number of elements in one of the odd rows and the even rows may be one less than the number of elements in the other.

Each element 23 is provided with an oscillating film 24. The outline of the oscillating film 24 is drawn by the dotted line in the plan view (plan view in the thickness direction of the substrate) in the direction perpendicular to the film surface of the oscillating film 24 in Fig. 3. A piezoelectric element 25 is formed on the oscillating film 24. The piezoelectric element 25 is configured from an upper electrode 26, a lower electrode 27, and a piezoelectric film 28. The piezoelectric film 28 is sandwiched between the upper electrode 26 and the lower electrode 27 in each element 23. These are layered in the order of lower electrode 27, the piezoelectric film 28, and the upper electrode 26. The ultrasonic device 17 is configured as one ultrasonic transducer element chip (substrate).

A plurality of first conductors 29 is formed on the front surface of the substrate 21. The first conductors 29 extend parallel to each other in the column direction in the array. One first conductor 29 is assigned to each element 23 in one column. One first conductor 29 is connected in common to the piezoelectric film 28 of the elements 23 aligned in the column direction of the array. The first conductor 29 forms an upper electrode 26 in each element 23. Both ends of a first conductor 29 are connected respectively to a pair of lead-out wires 31. The lead-out wires 31 extend parallel to each other in the row direction of the array. Consequently, all of the first conductors 29 have the same length. The upper electrode 26 is connected in common to the elements 23 in the entire matrix. For example, the first conductor 29 can be formed from iridium (Ir). However, other conductive materials may be used in the first conductor 29. The first conductor 29 and the lead-out wires 31 correspond to the wiring part formed by patterning a conductor layer that was deposited in layers.

A plurality of second conductors 32 is formed on a front surface of the substrate 21. The second conductors 32 extend parallel to each other in the row direction of the array. One second conductor 32 is assigned to each element 23 in one row. One second conductor 32 is arranged to share the piezoelectric film 28 of the elements 23 aligned in the row direction of the array. The second conductor 32 forms a lower electrode 27 in each element 23. For example, the second conductor 32 can be a layered film of titanium (Ti), iridium (Ir), platinum (Pt), and titanium (Ti). However, other conductive materials may be used in the second conductor 32. The second conductor 32 corresponds to the wiring part formed by patterning the conductive layer that was deposited as a layer.

The electrical conduction of an element 23 in each row is switched. A linear scan or a sector scan is implemented in response to the switching of this conduction. The count of the elements 23 in one row, namely the number of columns in the array, can be determined to correspond to the output level of the ultrasonic waves because the elements 23 in one row simultaneously output ultrasonic waves. For example, the number of columns may be set to approximately 10 to 15 columns. The truncated drawing shows 5 columns. The number of rows in the array can be determined in response to the width of the scanning range. For example, the number of rows may be set to 128 rows or 256 rows. The truncated drawing shows 8 rows. The roles of the upper electrode 26 and the lower electrode 27 may be switched. That is, the lower electrode may be connected in common to the elements 23 in the entire matrix, and the upper electrode may be connected to the elements 23 in common to each row of the array.

The outline of the substrate 21 has a first edge 21a and a second edge 21b that are a pair of mutually parallel lines that face each other and divide the outline. A first terminal array 33a in one line is arranged between the first edge 21a and the outline of the element array 22. A second terminal array 33b in one line is arranged between the second edge 21b and the outline of the element array 22. The first terminal array 33a can form one line parallel to the first edge 21a. The second terminal array 33b can form one line parallel to the second edge 21b. The first terminal array 33a is configured from a pair of upper electrode terminals 34 and a plurality of lower electrode terminals 35. Similarly, the second terminal array 33b is configured from a pair of upper electrode terminals 36 and a plurality of lower electrode terminals 37. The upper electrode terminals 34, 36 are connected respectively to the two ends of one lead-out wire 31. The lead-out wire 31 and the upper electrode terminals 34, 36 may be formed with plane symmetry in the perpendicular plane that bisects the element array 22. The lower electrode terminals 35, 37 are connected respectively to the two ends of one second conductor 32. The second conductor 32 and the lower electrode terminals 35, 37 may be formed with plane symmetry in the perpendicular plane that bisects the element array 22. Here, the outline of the substrate 21 is formed into a rectangle. The outline of the substrate 21 may be a square or a trapezoid.

A first flexible printed wiring board (hereinafter, referred to as the "first wiring board") 38 is connected to the substrate 21. The first wiring board 38 covers the first terminal array 33a. Conductive wires, namely first signal wires 39, are formed to correspond to each of the upper electrode terminals 34 and the lower electrode terminals 35 on one end of the first wiring board 38. The first signal wires 39 are separately matched opposite to and separately bonded to the upper electrode terminals 34 and lower electrode terminals 35. Similarly, a second flexible printed wiring board (hereinafter, referred to as the "second wiring board") 41 covers the substrate 21. The second wiring board 41 covers the second terminal array 33b. Conductive wires, namely second signal wires 42, are formed to correspond to each of the upper electrode terminals 36 and the lower electrode terminals 37 at one end of the second wiring board 41. The second signal wires 42 are separately matched opposite to and separately bonded to the upper electrode terminals 36 and lower electrode terminals 37.

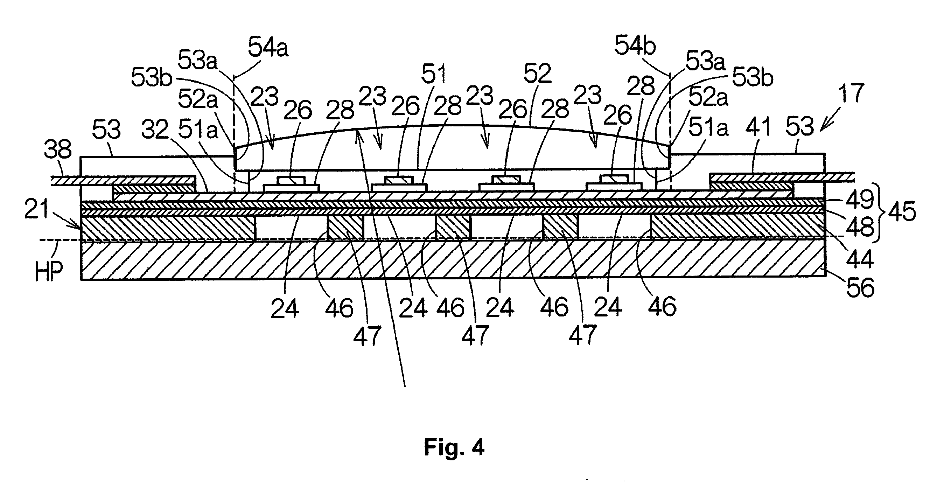

As shown in Fig. 4, the substrate 21 is provided with a substrate 44 and a coated film 45. The coated film 45 is formed on one side of the surface of the substrate 44. An opening 46 is formed in each element 23 in the substrate 44. The openings 46 are arranged in an array form on the substrate 44. The outline of the region in which the openings 46 are arranged corresponds to the outline of the element array 22. A partition wall 47 is delineated by two adjacent openings 46. Adjacent openings 46 are partitioned by a partition wall 47. The wall thickness of the partition wall 47 is equivalent to the interval between openings 46. The partition wall 47 specifies two wall surfaces in planes that extend parallel to each other. The wall thickness is equivalent to the distance between the two wall surfaces. That is, the wall thickness can be specified by the length of the perpendicular line sandwiched between the wall surfaces and perpendicular to the wall surfaces. For example, the substrate 44 may be formed from a silicon substrate.

The coated film 45 is composed of a silicon oxide (SiO₂) layer 48 that was deposited as a layer on the front surface of the substrate 44, and a zirconium oxide (ZrO₂) layer 49 that was deposited as a layer on the front surface of the silicon oxide layer 48. The coated film 45 is in contact with the openings 46. Thus, the portions of the coated film 45 that correspond to the outlines of the openings 46 form the oscillating film 24. The oscillating film 24 is a part of the coated film 45 that can oscillate the film in the thickness direction of the substrate 44 because it faces the openings 46. The film thickness of the silicon oxide layer 48 can be determined based on the resonance frequency.

The lower electrode 27, the piezoelectric film 28, and the upper electrode 26 are deposited as layers in order on the front surface of the oscillating film 24. The piezoelectric film 28 can be formed from, for example, lead zirconate titanate (PZT). Other piezoelectric materials may be used in the piezoelectric film 28. Here, the piezoelectric film 28 under the first conductor 29 completely covers the second conductor 32. Short circuits can be avoided between the first conductor 29 and the second conductor 32 by the function of the piezoelectric film 28.

An acoustic matching layer 51 is deposited as a layer on the front surface of the substrate 21. The acoustic matching layer 51 covers the element array 22. The film thickness of the acoustic matching layer 51 is determined to correspond to the resonance frequency of the oscillating film 24. For example, a silicone resin film can be used as the acoustic matching layer 51. The acoustic matching layer 51 is installed in the space between the first terminal array 33a and the second terminal array 33b. The edge of the acoustic matching layer 51 is separated from the first edge 21a and the second edge 21 b of the substrate 21. The acoustic matching layer 51 has an outline that is smaller than the outline of the substrate 21.

An acoustic lens 52 is arranged on the acoustic matching layer 51. The outline of the acoustic lens 52 is drawn further to the outside of the outline of the acoustic matching layer 51. Consequently, a space is formed between the acoustic lens 52 and the front surface of the substrate 21 in the periphery of the outline of the acoustic matching layer 51. The edge of the acoustic lens 52 is separated from the first edge 21a and the second edge 21b of the substrate 21. The acoustic lens 52 adheres to the front surface of the acoustic matching layer 51. The acoustic lens 52 is affixed to the substrate 21 by the function of the acoustic matching layer 51. The outer front surface of the acoustic lens 52 is formed from a partial cylindrical surface. The partial cylindrical surface has a generatrix parallel to the first conductor 29. The curvature of the partial cylindrical surface is determined to correspond to the focus position of ultrasonic waves generated by the elements 23 in one row that is connected to one line of second conductors 33. For example, the acoustic lens 52 is formed from silicone resin. The acoustic lens 52 has an acoustic impedance that is close to the acoustic impedance of a living body.

A protective film (structure) 53 is fixed to the substrate 21. The protective film 53 is formed from a raw material having the waterproofing property, for example, an epoxy resin. However, the protective film 53 may be formed from other resin materials. The protective film 53 has larger compressive strength in at least the direction perpendicular to the front surface of the substrate 21 than the compressive strength of the acoustic matching layer 51. The protective film 53 is sandwiched between the acoustic lens 52 and the substrate 21 in the periphery of the acoustic matching layer 51. Generally, the compressive strength of the silicone resin is from roughly 58.8 MPa to less than 98.06 MPa. Because the compressive strength of an epoxy resin is from roughly 98.06 MPa to 196.12 MPa, a protective film 53 of epoxy resin has a larger compressive strength than that of the acoustic matching layer 51 of silicone resin. The compressive strength was measured in accordance with Japanese Industrial Standards (JIS) K718.

Here, the protective film 53 is fixed to the side surfaces 52a, 51a of the acoustic lens 52 and the acoustic matching layer 51. Side surfaces 52a, 51a extend perpendicular to the front surface of the substrate 21. In Fig. 4, the protective film 53 sandwiches the acoustic matching layer 51 and the acoustic lens 52 respectively at the contact surfaces 53a, 53b respectively along the two virtual planes 54a, 54b that extend parallel to the generatrix of the acoustic lens 52 and intersect perpendicular to the substrate 21. The protective film 53 covers the second conductor 32 and the lead-out wires 31 of the substrate 21 between the acoustic matching layer 51 and the first and second wiring boards 38, 41. Similarly, the protective film 53 covers the ends of the first wiring board 38 and the second wiring board 41 on the substrate 21.

A backing material 56 is fixed to the back surface of the substrate 21. The back surface of the substrate 21 is stacked on the front surface of the backing material 56. The backing material 56 closes the opening 46 on the back surface of the ultrasonic device 17. The backing material 56 can be provided by a rigid base material. Here, the partition wall 47 is bonded to the backing material 56. The backing material 56 is bonded to at least one bonding region in each partition wall 47. An adhesive agent can be used in the bonding.

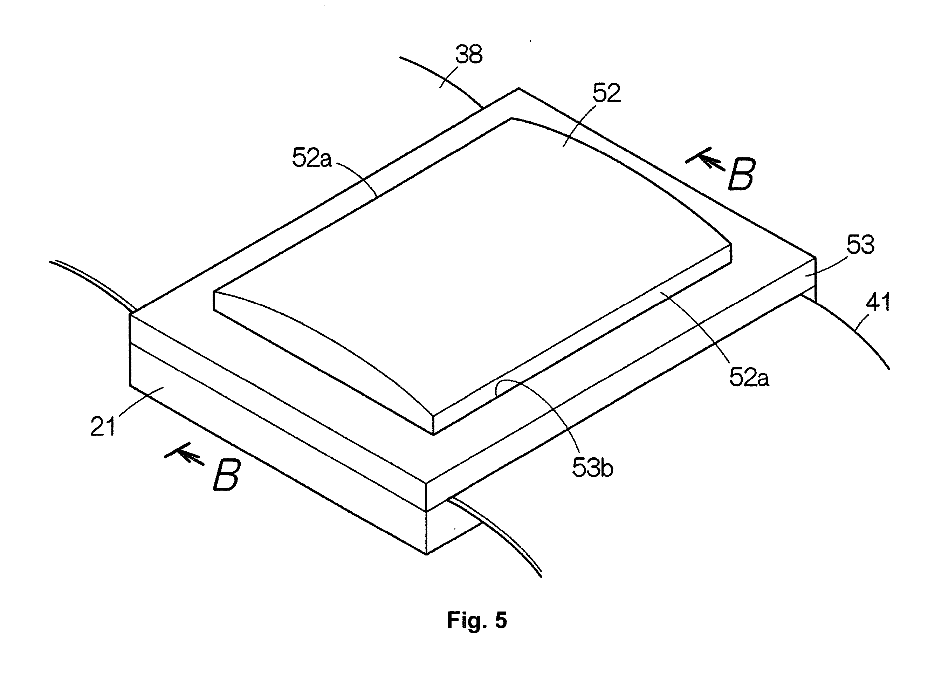

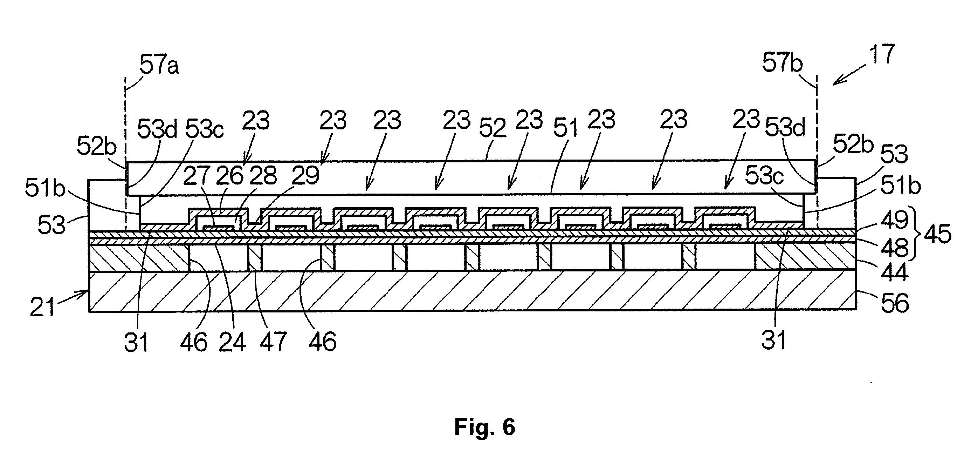

As shown in Fig. 5, the protective film 53 encloses the acoustic lens 52 along the outline of the acoustic lens 52 in a plan view. The protective film 53 extends in a frame shape between the outline of the acoustic lens 52 and the peripheral edge of the substrate 21. As shown in Fig. 6, the protective film 53 is fixed to the side surfaces 52b, 51 b of the acoustic lens 52 and acoustic matching layer 51 to form the frame shape. The side surfaces 52b, 51b are connected perpendicular to the side surfaces 52b, 51b that rise from the front surface of the substrate 21 perpendicular to the surface of the substrate 21. The protective film 53 sandwiches the acoustic matching layer 51 and the acoustic lens 52 respectively at the contact surfaces 53c, 53d respectively along the two virtual planes 57a, 57b that intersect perpendicular to the generatrix of the acoustic lens 52 and intersect perpendicular to the substrate 21.

### (2) Operation of Ultrasound Diagnostic Apparatus

Next, the operation of the ultrasound diagnostic apparatus 11 is briefly explained. A pulse signal is supplied to the piezoelectric element 25 in the transmission of ultrasonic waves. The pulse signal is supplied to the elements 23 in each row through the lower electrode terminals 35, 37 and the upper electrode terminals 34, 36. In each element 23, an electric field acts on the piezoelectric film 28 between the lower electrode 27 and the upper electrode 26. The piezoelectric film 28 oscillates at the frequency of the ultrasonic waves. The oscillation of the piezoelectric film 28 is transmitted to the oscillating film 24. The oscillating film 24 oscillates ultrasonic waves. As a result, the desired ultrasound beam is emitted toward the target object (e.g., interior of the human body).

The reflected waves of the ultrasonic waves oscillate the oscillating film 24. The ultrasound oscillation of the oscillating film 24 is ultrasound oscillation of the piezoelectric film 28 at the desired frequency. Voltage is output from the piezoelectric element 25 in response to the piezoelectric effect of the piezoelectric element 25. A voltage potential is generated between the upper electrode 26 and the lower electrode 27 in each element 23. The voltage potential is output as electrical signals from the lower electrode terminals 35, 37 and the upper electrode terminals 34, 36. Thus, the ultrasonic waves are detected.

The transmission and reception of the ultrasonic waves are repeated. As a result, a linear scan and a sector scan are implemented. When the scan is completed, the image is formed based on digital signals of the output signals. The formed image is displayed on the screen of the display panel 15.

The acoustic lens 52 presses against the living body to form an ultrasound image. When the acoustic lens 52 presses against the living body, the pressing force toward the substrate 21 acts on the acoustic lens 52. Because the protective film 53 has larger compressive strength than the compressive strength of the acoustic matching layer 51, the acoustic lens 52 is stopped by the protective film 53 in the direction of the pressing force. Even when the pressing force is applied to the acoustic lens 52, deformation of the acoustic lens 52 can be prevented. Deformation of the acoustic lens 52 can be adequately suppressed for the pressing force in the ultrasonic device 17.

As described above, the partial cylindrical surface formed by the generatrix parallel to one center axis in the acoustic lens 52 is specified. The partial cylindrical surface is used in the convergence of the ultrasonic waves. The focus position of the ultrasonic waves is determined at the partial cylindrical surface. Because a contact surface 53a of the protective film 53 sandwiches the acoustic matching layer 51 in the direction that intersects perpendicular to the generatrix, the acoustic lens 52 is supported by the protective film 53 along the outline parallel to the generatrix of the acoustic lens 52. The acoustic lens 52 is longer in the direction parallel to the generatrix than that in the direction intersecting the generatrix. Consequently, an interval is sandwiched between a pair of protective films 53 in the direction that intersects perpendicular to the generatrix. Thus, deformation of the acoustic lens 52 is effectively suppressed. The focus position of the acoustic lens 52 can be maintained at the determined position. There is the risk that if deformation is produced in the acoustic lens 52, an offset will arise in the focus position of the acoustic lens 52. When the focus position is offset, a precise ultrasound image cannot be drawn. Moreover, in this embodiment, the protective film 53 sandwiches the acoustic matching layer 51 by the contact surface 53c in the direction along the generatrix of the acoustic lens 52. The acoustic lens 52 is supported by the protective film 53 along the outline in the direction that intersects the generatrix of the acoustic lens 52. Thus, deformation of the acoustic lens 52 is more effectively suppressed. In particular, the protective film 53 surrounds the acoustic matching layer 51 along the front surface of the substrate 21. Thus, the acoustic matching layer 51 is sealed by the front surface of the substrate 21, the acoustic lens 52, and the protective film 53. Because movement of the acoustic matching layer 51 is prevented by the sealed spaces, crushing of the acoustic matching layer 51 is prevented even when a pressing force acts on the acoustic matching layer 51. Deformation of the acoustic matching layer 51 is prevented. The acoustic lens 52 is effectively supported by the protective film 53 and the acoustic matching layer 51. Deformation of the acoustic lens 52 is suppressed.

In the ultrasonic device 17, the element array 22 and the first and second wiring boards 38, 41 are electrically connected to each other by a second conductor 33 and lead-out wires 31 on the substrate 21. The protective film 53 covers a second conductor 32 and the lead-out wires 31 on the substrate 21 between the acoustic matching layer 51 and the first and second wiring boards 38, 41. Because the second conductor 33 and the lead-out wires 31 are covered by the protective film 53 between the acoustic matching layer 51 and the first and second wiring boards 38, 41, exposure of the second conductor 33 and the lead-out wires 31 is avoided. Thus, the conductors on the substrate 21 are protected. In particular, when the waterproofing property is given to the protective film 53, the second conductor 33 and the lead-out wires 31 are protected from moisture and humidity, and short circuits can be prevented between conductors such as the second conductor 33 and the lead-out wires 31. As described earlier, when the protective film 53 covers the ends of the first and second wiring boards 38, 41, the protective film 53 can reinforce the fixing strength of the first and second wiring boards 38, 41.

### (3) Manufacturing Method of Ultrasonic Device

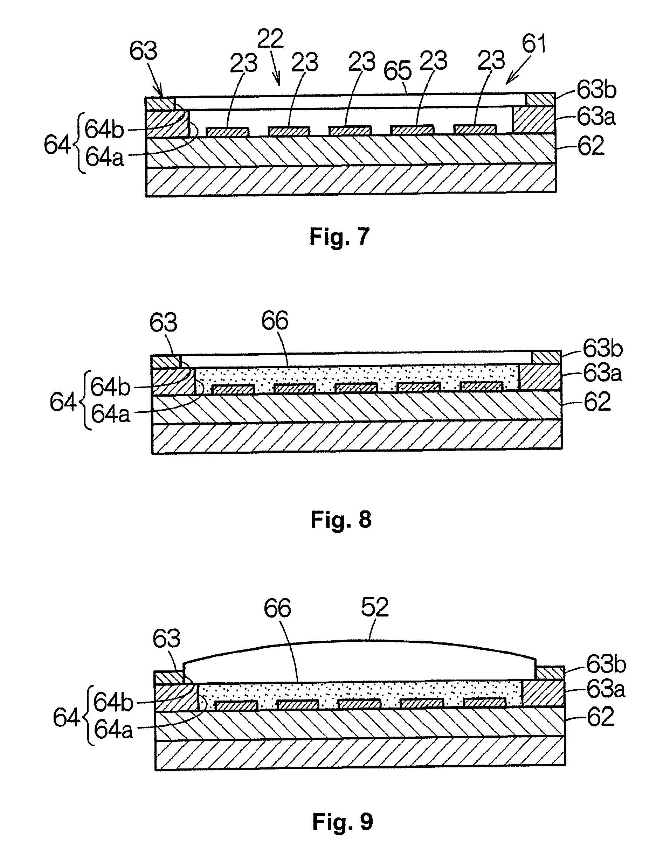

Next, the manufacturing method of the ultrasonic device 17 is explained. As shown in Fig. 7, a substrate 61 is prepared. The substrate 61 has an element array 22 that includes a plurality of elements 23 arranged in an array form on a base material 62. The base material 62 corresponds to the substrate 21 described above. A masking material 63 is deposited as a layer on the base material 62. The masking material 63 is formed into a shape that encloses the space between the outline of the element array 22 and the peripheral edge of the base material 62 in the plan view. The masking material 63 forms an opening 64 on the element array 22. The opening 64 delineates the frame surrounding the element array 22 on the base material 62. Thus, the masking material 63 is arranged on both sides of the outline of the element array 22 in the extension direction of the first conductor 29 simultaneous to being placed on both sides of the outline of the element array 22 in the extension direction of the second conductor 33 in the plan view. Here, the masking material 63 is formed in a 2-layer layered structure. The thickness of the lower layer 63a is identical to the film thickness of the acoustic matching layer 51. The frame formed by the lower layer 63a delineates a lower opening 64a. An upper layer 63b delineates an upper opening 64b that extends further to the outside than the outline of the lower opening 64a in the plan view. Thus, a step difference is formed between the lower opening 64a and the upper opening 64b. For example, a photoresist can be used as the masking material 63.

Next, the acoustic matching layer 51 and the acoustic lens 52 are arranged on the element array 22 in the opening 64 of the masking material 63. In this arrangement, as shown in Fig. 8, a resin material 66 of the acoustic matching layer 51 is poured into the opening 64 of the masking material 63. The resin material 66 has fluidity. The resin material 66 fills the lower opening 64a of the lower layer 63a. The flow of the resin material 66 is dammed by the lower layer 63a of the masking material 63. Thus, the form of this acoustic matching layer 51 is prepared. The resin material 66 spreads uniformly inside the lower opening 64a of the lower layer 63a. Here, the volume of resin material 66 is adjusted based on the thickness of the lower layer 63a. The thickness of the resin material 66 can be controlled with high precision by the function of the thickness of the lower layer 63a.

As shown in Fig. 9, the acoustic lens 52 is stacked on the resin material 66 in the opening 64 of the masking material 63. The lower layer 63a of the masking material 63 is sandwiched between the acoustic lens 52 and the base material 62. The acoustic lens 52 may be finished to a predetermined shape. The upper layer 63b of the masking material 63 positions the acoustic lens 52 from both sides in the extension direction of the second conductor 33 in the plan view, and simultaneously positions the acoustic lens 52 from both sides in the extension direction of the first conductor 29. Thus, the upper layer 63b of the masking material 63 implements positioning from four directions with respect to the acoustic lens 52 in the upper opening 64b. The acoustic lens 52 can be positioned with respect to the element array 22 with high precision by the function of the upper layer 63b of the masking material 63. Here, the edge of the acoustic lens 52 is in contact with the wall surface of the upper layer 63b. The resin material 66 hardens (cures) in response to irradiation with heat or ultraviolet light. The resin material 66 hardens to form the acoustic matching layer 51. This acoustic lens 52 adheres to the element array 22.

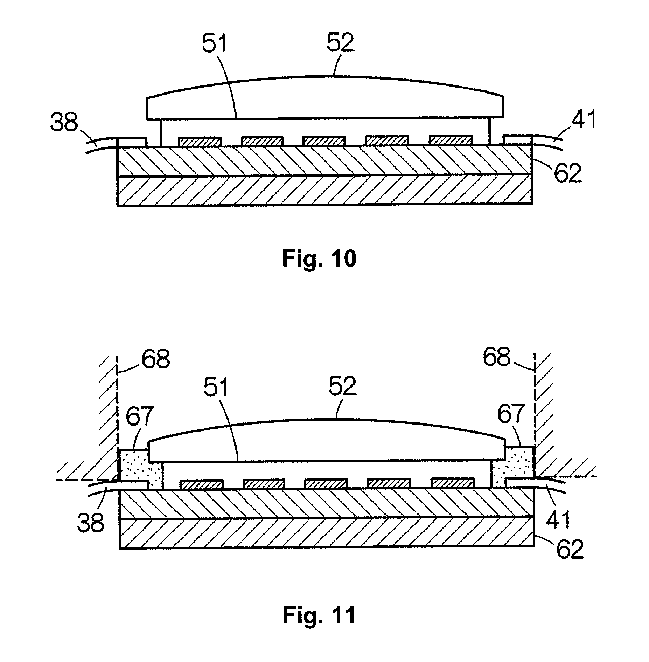

As shown in Figure 10, when the acoustic matching layer 51 hardens, the masking material 63 is removed. For example, the masking material 63 may be removed by an etching process or another process. The space between the acoustic lens 52 and the base material 62 is delineated by the function of the step difference of the lower layer 63a.

When the masking material 63 is removed, the first terminal array 33a and the second terminal array 33b are exposed between the outline of the element array 22 and the peripheral edge of the base material 62 on the front surface of the base material 62. After the masking material 63 is removed, the first wiring board 38 and the second wiring board 41 are bonded to the outline of the element array 22 and the peripheral edge of the base material 62, respectively. The first wiring board 38 covers the first terminal array 33a. The second wiring board 41 covers the second terminal array 33b. The first signal wires 39 of the first wiring board 38 are separately connected to the upper electrode terminal 34 and the lower electrode terminal 35. The second signal wires 42 of the second wiring board 41 are separately connected to the upper electrode terminal 36 and the lower electrode terminal 37. Other bonding methods may be used for bonding.

As shown in Fig. 11, a protective film 53 is formed on the base material 62. Resin material 67 is filled between the acoustic matching layer 51 and the first wiring board 38, and between the acoustic matching layer 51 and the second wiring board 41. For example, a nozzle may be used to supply the resin material 67. The nozzle may move along the edge of the first wiring board 38 and the edge of the second wiring board 41 parallel to the generatrix of the acoustic lens 52. The resin material 67 has fluidity. An enclosure 68 may be delineated on the first wiring board 38 and the second wiring board 41 for the pouring in of the resin material 67. For example, the enclosure 68 can be formed from metal. The enclosure 68 can stop the flow of the resin material 67. The resin material 67 hardens in response to the irradiation of heat or ultraviolet light. The resin material 67 hardens to form the protective film 53. An epoxy resin may be used as resin material 66 and resin material 67. In this case, the acoustic lens 52 may be formed from an epoxy resin.

### (4) Ultrasonic Device Related to Modified Example

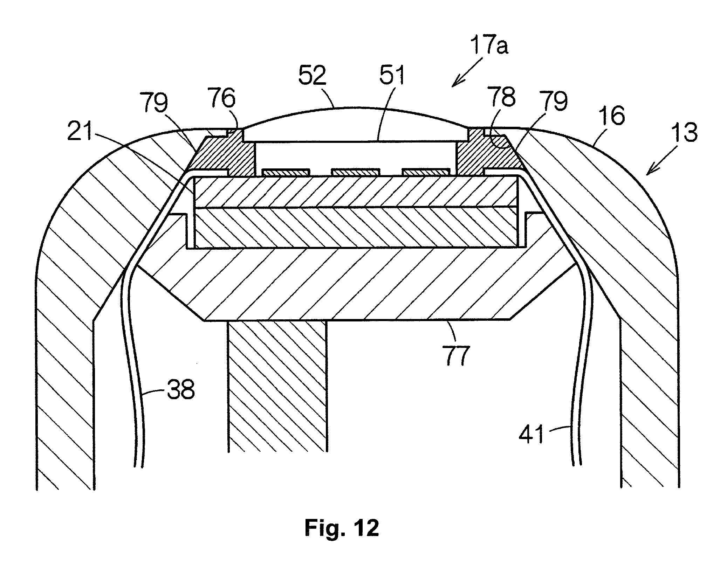

Fig. 12 schematically shows an ultrasonic device 17a related to a first modified example. The case 16 of the ultrasonic probe 13 is formed with an opening 76. The acoustic lens 52 is arranged in the opening 76. The ultrasonic device 17a is supported by a support part 77. The support part 77 is bonded to the inside of the case 16. In the bonded, the first wiring board 38 and the second wiring board 41 are sandwiched between the support part 77 and the case 16. Spaces 78 are delineated between the substrate 21 and the case 16 in the surroundings of the acoustic lens 52 and the acoustic matching layer 51. The spaces 78 are filled with a protective material 79. The protective material 79 is sandwiched between the acoustic lens 52 and the substrate 21. The protective material 79 has a larger compressive strength than the compressive strength of the acoustic matching layer 51. The protective material 79 fixes the acoustic lens 52, the acoustic matching layer 51, and the substrate 21 in the case 16. The protective material 79 can function similar to the protective film 53 described above. The other structures are similar to those in the ultrasonic device 17 described above.

In the manufacture of the ultrasonic probe 13, the ultrasonic device 17a and the support part 77 are housed in the case 16. When the support part 77 is fixed to the case 16, the acoustic lens 52 faces the opening 76. In the surroundings of the acoustic lens 52 and the acoustic matching layer 51, the spaces 78 are delineated between the substrate 21 and the case 16. A resin material having fluidity fills the spaces 78 from the gap of the opening 76. When the resin material hardens, the protective material 79 is formed. The protective material 79 prevents movement of the acoustic lens 52 in the opening 76. The acoustic lens 52 is reliably fixed in the case 16.

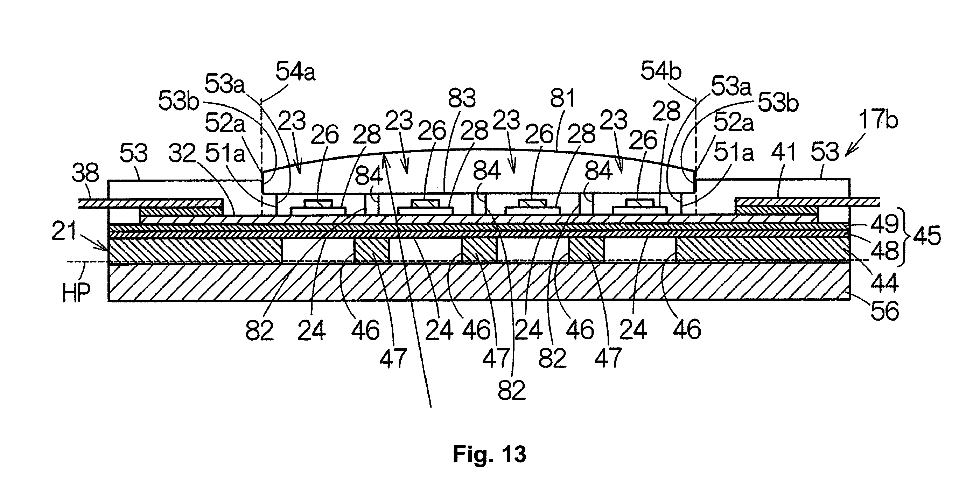

Fig. 13 schematically shows an ultrasonic device 17b related to a second modified example. A columnar part 82 that projects out toward the substrate 21 from the surface facing the substrate 21 is formed in an acoustic lens 81. On the other side, a groove (depression) 84 that engages the columnar part 82 is formed in an acoustic matching layer 83. The groove 84 can pass through the acoustic matching layer 83. The groove 84 defines an inner surface of the acoustic matching layer 83 that is in contact with the columnar part 82. In this case, the tip of the columnar part 82 is in contact with the substrate 21. According to this structure, offsets between the acoustic lens 81 and the acoustic matching layer 83 are prevented in the shear direction along the boundary surface of the acoustic lens 81 and the acoustic matching layer 83. The acoustic lens 81 and the acoustic matching layer 83 are rigidly layered together to form a layered structure. This layered structure further enhances the suppression of deformation of the acoustic lens 81. The other structures are similar to those of the ultrasonic device 17 described above. In addition, similar to the ultrasonic device 17a described above, in the ultrasonic device 17b, there is a protective material instead of the protective film 53, and the acoustic lens 81, the acoustic matching layer 83, and the substrate 21 may be fixed to the case 16 of the ultrasonic probe 13.

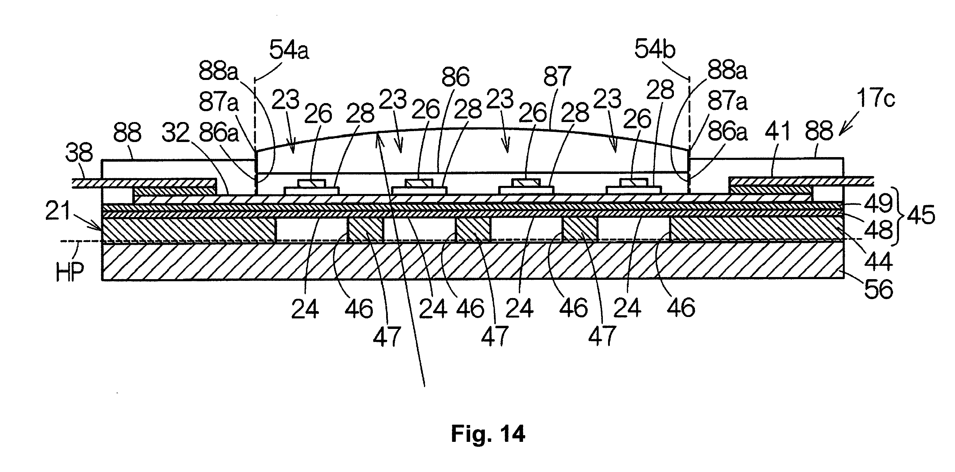

Fig. 14 schematically shows an ultrasonic device 17c related to a third modified example. An acoustic matching layer 86 covers the element array 22. The acoustic matching layer 86 has a first compressive strength. The acoustic matching layer 86 is a single layer and has a film thickness that is 100 µm or less and an acoustic impedance of 1 MRayls or less. Here, the acoustic matching layer 86 is formed from epoxy resin. An acoustic lens 87 is arranged on the acoustic matching layer 86. Similar to the above description, the acoustic lens 87 has a second compressive strength that is larger than the first compressive strength of the acoustic matching layer 86. Thus, the acoustic lens 87 can be formed from silicone resin. In the plan view, the outline of the acoustic lens 87 can coincide with the outline of the acoustic matching layer 86.

A protective film (structure) 88 is fixed to the substrate 21. For example, the protective film 88 is formed from raw materials having the waterproofing property, such as epoxy resin. However, the protective film 88 may be formed from other resin materials. The protective film 88 is in contact with the acoustic lens 87 and the acoustic matching layer 86. Here, the protective film 88 is sandwiched by the acoustic lens 87 and the acoustic matching layer 86 at contact surfaces 88a respectively along the two virtual planes 54a, 54b that expand parallel to the generatrix of the acoustic lens 87 and intersect perpendicular to the substrate 21. Side surfaces 87a, 86a of the acoustic lens 87 and the acoustic matching layer 86 spread out in one plane. The protective film 88 covers the second conductor 32 and the lead-out wires 31 on the front surface of the substrate 21 between the acoustic matching layer 86 and the first and second wiring boards 38, 41. Similarly, the protective film 88 covers the ends of the first wiring board 38 and the second wiring board 41 on the substrate 21. The other structures are similar to ultrasonic device 17 described above. Similar to the ultrasonic device 17a described above, in the ultrasonic device 17c, there is a the protective material instead of the protective film 88, and the acoustic lens 87, the acoustic matching layer 86, and the substrate 21 may be fixed to the case 16 of the ultrasonic probe 13.

When the acoustic lens 87 is pressed against a living body, a pressing force directed toward the substrate 21 acts on the acoustic lens 87. At this time, the acoustic lens 87 is stopped by the acoustic matching layer 86 in the direction of the pressing force. Compared to when the acoustic matching layer 86 has a compressive strength equivalent to that of the acoustic lens 87, crushing of the acoustic matching layer 86 is prevented. Even if a pressing force is applied to the acoustic lens 87, deformation of the acoustic lens 87 can be prevented. In the ultrasonic device 17c, deformation of the acoustic lens 87 can be adequately suppressed for the pressing force. Because the acoustic matching layer 86 is one layer, compared to when the acoustic matching layer 86 is formed from a plurality of layers, the formation step of the acoustic matching layer 86 can be simplified. Moreover, the film thickness of the acoustic matching layer 86 can be controlled with high precision. Here, the acoustic impedance of the acoustic matching layer 86 is 2 MRayls or less, a match of the acoustic impedance can be established between the acoustic lens 87 and the element 23. For example, the acoustic matching layer 86 is a thinner film compared to bulk transducer elements. The film thickness of the acoustic matching layer 86 can be fabricated as a thin film of 100 µm or less, and deformation of the acoustic matching layer 86 is suppressed in response to the thinning of the film. In addition, the ultrasonic device 17c is reduced in size.

This embodiment was explained in detail above, but the possibility of many modifications that do not essentially deviate from the novel items and the effects of the present invention can be readily understood by a person skilled in the art. Thus, these kinds of modified examples are included in the scope of the present invention. For example, a term cited at least once with a term that has a broader meaning or a different meaning in the specification or the drawings can be replaced with that different term in all locations in the specification or the drawings. In addition, structures such as the ultrasound diagnostic apparatus 11; the apparatus terminal 12; the ultrasonic probe 13; the display panel 15; the case 16; the substrate 21; the element 23; the first and second wiring boards 38, 41; the acoustic matching layers 51, 83, 86; and the acoustic lenses 52, 81, 87, and the operations thereof are not limited to those explained in this embodiment and can have various modifications.

### GENERAL INTERPRETATION OF TERMS

In understanding the scope of the present invention, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives. Also, the terms "part," "section," "portion," "member" or "element" when used in the singular can have the dual meaning of a single part or a plurality of parts. Finally, terms of degree such as "substantially", "about" and "approximately" as used herein mean a reasonable amount of deviation of the modified term such that the end result is not significantly changed. For example, these terms can be construed as including a deviation of at least ± 5% of the modified term if this deviation would not negate the meaning of the word it modifies.

While only selected embodiments have been chosen to illustrate the present invention, it will be apparent to those skilled in the art from this disclosure that various changes and modifications can be made herein without departing from the scope of the invention as defined in the appended claims. Furthermore, the foregoing descriptions of the embodiments according to the present invention are provided for illustration only, and not for the purpose of limiting the scope of the invention as defined by the appended claims.

## Claims

1. An ultrasonic device (17) comprising:
a substrate (21) having an element array (22) including a plurality of thin-film ultrasonic transducer elements (23) arranged in an array form;
an acoustic matching layer (51, 83) covering the element array (22);
an acoustic lens (52, 81) arranged on the acoustic matching layer (51, 83); and
a structure (53, 79) that is arranged between the acoustic lens (52, 81) and the substrate (21) and has a larger compressive strength than a compressive strength of the acoustic matching layer (51, 83).

2. The ultrasonic device (17) according to claim 1, wherein the structure (53, 79) sandwiches the acoustic matching layer (51, 83) by two side surfaces in contact respectively with side surfaces of the acoustic matching layer (51, 83) extending along a direction parallel to a generatrix of the acoustic lens (52, 81), or extending along a direction intersecting the generatrix of the acoustic lens (52, 81).

3. The ultrasonic device (17) according to claim 1 or 2, wherein the structure (53, 79) encloses the acoustic matching layer (51, 83) along a surface of the substrate (21).

4. The ultrasonic device (17) according to any one of claims 1 to 3, wherein
the acoustic lens (81) has a columnar part (82) projecting out toward the substrate (21) from a surface facing the substrate (21), and
a through hole (84) is formed in the acoustic matching layer (83), and the through hole (84) has an inner surface in contact with the columnar part (82).

5. The ultrasonic device (17) according to any one of the preceding claims, further comprising a flexible wiring board (38, 41) bonded to the substrate (21) on an outside of an outline of the acoustic matching layer (51, 83) in a plan view along a thickness direction of the substrate (21), wherein the structure (53, 79) covers a conductor on the substrate (21) between the acoustic matching layer (51, 83) and the flexible wiring board (38, 41), wherein the structure (53, 79) is preferably arranged on the flexible printed wiring board (38, 41).

6. A probe (13) comprising:
the ultrasonic device (17) according to any one of the preceding claims; and
a case (16) supporting the ultrasonic device (17), wherein the structure (53, 79) is preferably fixed in the case (16).

7. An electronic device comprising:
the ultrasonic device (17) according to any one of claims 1 to 5; and
a processing unit (12) connected to the ultrasonic device (17), and configured to process an output of the ultrasonic device (17).

8. An ultrasound imaging apparatus (11) comprising:
the ultrasonic device (17) according to any one of claims 1 to 5;
a processing unit (12) connected to the ultrasonic device (17), and configured to process an output of the ultrasonic device (17) and to generate an image; and
a display device (15) configured and arranged to display the image.

9. An ultrasonic device (17) comprising:
a substrate (21) having an element array (22) including a plurality of thin-film ultrasonic transducer elements (23) arranged in an array form;
an acoustic matching layer (86) covering the element array (22) and having a first compressive strength; and
an acoustic lens (87) that is arranged on the acoustic matching layer (86) and has a second compressive strength that is smaller than the first compressive strength.

10. The ultrasonic device (17) according to claim 10, wherein the acoustic matching layer (86) is a single layer,
wherein an acoustic impedance of the acoustic matching layer (86) is preferably 2 MRayls or less,
and/or wherein a film thickness of the acoustic matching layer (86) is preferably 100 µm or less.

11. A manufacturing method of an ultrasonic device (17) comprising:
arranging a masking material on a substrate (21) having an element array (22) including a plurality of thin-film ultrasonic transducer elements (23) arranged in an array form so that the masking material is arranged on both sides of a region of the substrate (21) where the element array (22) is arranged in a plan view along a thickness direction of the substrate (21);
arranging an acoustic matching layer (51, 83) and an acoustic lens (52, 81) on the element array (22) between the masking material so that the masking material is sandwiched between the acoustic lens (52, 81) and the substrate (21);
removing the masking material; and
forming a structure (53, 79) between the acoustic lens (52, 81) and the substrate (21), the structure (53, 79) having a larger compressive strength than a compressive strength of the acoustic matching layer (51, 83).

12. The manufacturing method of an ultrasonic device (17) according to claim 11, further comprising:
bonding a flexible printed wiring board (38, 41) to the substrate (21) at a position between an edge of the region and an edge of the substrate (21) on both sides of the region, after the removing of the masking material, wherein
the forming of the structure (53, 79) includes pouring a resin material having fluidity between the acoustic matching layer (51, 83, 86) and the flexible printed wiring board (38, 41) and curing the resin material to form the structure (53, 79).

13. The manufacturing method of an ultrasonic device (17) according to claim 11 or 12, wherein the arranging of the acoustic lens (52, 81) includes positioning the acoustic lens (52, 81) using the masking material contacting the acoustic lens (52, 81) from both sides of the acoustic lens (52, 81).

14. The manufacturing method of an ultrasonic device (17) according to claim 13, wherein the arranging of the acoustic lens (52, 81) includes positioning the acoustic lens (52, 81) using the masking material having an opening in which the acoustic lens (52, 81) is placed so that the masking material positions the acoustic lens (52, 81) in four directions.

15. The manufacturing method of an ultrasonic device (17) according to claim 14, wherein the arranging of the acoustic matching layer (51, 83) includes pouring resin material having fluidity into the opening, and controlling a thickness of the resin material by a thickness of the masking material.
